# EUROPÄISCHE PATENTSCHRIFT

(11) **EP 0 739 233 B1**
(45) Veröffentlichungstag und Bekanntmachung des Hinweises auf die Patenterteilung: **23.09.1998**
(21) Anmeldenummer: 95905045.1
(22) Anmeldetag: 10.01.1995
(51) Int. Cl.: B01D 59/40, G21G 1/10

(54) **VERFAHREN ZUR ABTRENNUNG VON TRÄGERFREIEN RADIONUKLIDEN AUS TARGETFLÜSSIGKEIT, DESSEN ANWENDUNG UND DAFÜR GEEIGNETE ANORDNUNG**
PROCESS FOR SEPARATING CARRIER-FREE RADIONUCLIDES FROM TARGET LIQUIDS, ITS USE AND ARRANGEMENT THEREFOR
PROCEDE DE SEPARATION DE RADIONUCLIDES SANS ENTRAINEURS CONTENUS DANS UN LIQUIDE CIBLE, SON UTILISATION ET DISPOSITIF DE MISE EN OEUVRE DE CE PROCEDE

(30) Priorität: 11.01.1994 DE 4400539
(43) Veröffentlichungstag der Anmeldung: 30.10.1996
(73) Patentinhaber: FORSCHUNGSZENTRUM JÜLICH GMBH, 52425 Jülich (DE)
(72) Erfinder: HAMACHER, Kurt, D-52076 Aachen (DE); BLESSING, Gerrit, D-52072 Aachen (DE)
(86) Internationale Anmeldenummer: DE9500025
(87) Internationale Veröffentlichungsnummer: WO9518668

(56) Entgegenhaltungen:
- APPLIED RADIATION AND ISOTOPES , Bd. 40,Nr. 1, 1989 Seite 1-6 ALEXOFF 'recovery of (18 f ) fluoride from (18 o) water in an electrochemical cell' in der Anmeldung erwähnt
- DATABASE INSPEC INSTITUTE OF ELECTRICAL ENGINEERS, STEVENAGE, GB Inspec No. an-2586186, 1985 WEINREICH ET AL 'radiopharmaceuticals and labelled compounds' & 'Proceedings of an IAEA international conference , Tokyo, Japan, 1984'
- APPL. RADIAT. ISOT., Bd. 42,Nr. 12, 1991 UK, Seiten 1231-1233, SUEHIRO ET AL 'A practical production of no-carrier-added F18 from natural water etc...'
- APPLIED RADIATION AND ISOTOPES, Bd. 37,Nr. 7, 1986 UK, Seiten 631-631-632, KEINONEN ET AL. 'effective small volume (18 O) target for the production of (18 F) fluoride.'

## Beschreibung

Die Erfindung bezieht sich auf ein Verfahren zur Abtrennung von trägerfreien Radionukliden aus flüssigem bzw. verflüssigbarem Targetmaterial mit geringer elektrischer Leitfähigkeit, die durch einen Nuklearprozeß, insb. durch Zyklotronstrahlung, in ionisierbarer bzw. polarisierbarer Form entstehen, innerhalb einer Elektrodenanordnung durch Anlegen eines elektrischen Feldes zur Elektrofixierung des Nuklids an einer der Elektroden, von der das Nuklid nach Entfernung der Targetflüssigkeit und Zugabe von Lösungmittel für die weitere Verarbeitung bei abgeschaltetem Feld oder in einem Feld entgegengesetzter Polung wieder in Lösung gebracht wird, und sie umfaßt die Anwendung dieses Verfahrens und eine dafür geeignete Durchflußzelle.

Bei der Herstellung von Radiotracer-Verbindungen, die insbesondere in der medizinischen Diagnostik eine bedeutende Rolle spielen, werden insbesondere kurzlebige Radionuklide eingesetzt, die ferner möglichst in trägerfreier Form vorgesehen werden.

Daraus ergibt sich ein Zwang zu möglichst zeitsparender Arbeitsweise mit möglichst hoher Markierungsausbeute trotz trägerfreier Arbeitsweise, wobei zusätzlich bei Verwendung teurer Targetmaterialien auf deren möglichst verlustarme Rückgewinnung geachtet werden sollte.

Ein besonders elegantes Verfahren zur Gewinnung von Radionukliden für die Radiotracertechnik besteht in der Zyklotron-Bestrahlung von Targetmaterial, wofür als Beispiel zur Veranschaulichung die Erzeugung von ¹⁸F aus ¹⁸O-H₂O dienen kann: ¹⁸F hat eine Halbwertszeit von ca. 110 min und wird u.a. zur Synthese von ¹⁸F-Fluordesoxyglucose für die Positronen-Emissions-Tomographie angewandt. Es wird durch Zyklotron-Bestrahlung aus ¹⁸O-H₂O gebildet, das mit einem Preis von etwa 150,-- DM/g in die Prozeßkosten eingeht.

Gemäß der bereits seit etwa 8 Jahren umfänglich angewandten Technik wird das im Zyklotron gebildete ¹⁸F-Fluorid vom ¹⁸O-H₂O durch Ionenaustausch getrennt, wobei zum einen [¹⁸O]H₂O-Verluste sowie eine Kontamination desselben durch organische Inhaltsstoffe der Ionenarnstauscher und [¹⁶O]H₂O-Zumischung durch anhängende Feuchtigkeit auftreten.

Daneben ist auch eine Verfahrensweise bekannt, nach der die Targetflüssigkeit unter Zusatz von basischen Phasentransferkatalysatoren und/oder von Carbonaten vom [¹⁸F]-Fluorid durch Destillation getrennt wird.

Die vorstehenden Verfahrensweisen sind klar ersichtlich unbefriedigend, obzwar bereits über längere Zeit im Gebrauch.

Es wurde auch bereits ein Verfahren der eingangs genannten Art von D.Alexoff et al. (Appl.Radiat.Isot. Vol.40, No.1, Seiten 1-6, 1989) untersucht, die eine elektrochemische Zelle aus ineinandergreifenden Tiegeln mit veränderbarem Abstand beschreiben, von denen der innere, als Kathode dienende Platintiegel mittels einer Mikrometerschraube höhenverstellbar und der äußere aus Graphit oder Glaskohlenstoff bestehende Tiegel in einer zerlegbaren PVC-Halterung untergebracht und für einen Flüssigkeitsaustausch über eine Art Heber eingerichtet ist.
Die Gleichspannungsquelle war von 0 bis 20V regulierbar bei Stromstärken bis 1 Ampère. Soweit ersichtlich, wurden Feldstärken von 100 - 300 V/cm angewandt.

Für die Verwendung von Graphittiegeln werden ¹⁸F-Ausbeuten bis 70% angegeben. Insgesamt werden Zellen mit Platin- und Glaskohlenstoffelektroden als ineffizient für Routineproduktion größerer Nuklidmengen bezeichnet und der Ionenaustauscher-Methode der Vorzug gegeben.

Entgegen dieser Auffassung wurde nun jedoch eine der Praxis besonders angemessene Radionuklidabtrennung vom Targetmaterial unter praktisch verlustfreier Rückgewinnung des letzteren und ausreichend hoher Ausbeute an Radionuklid bei geringem Zeitaufwand entwickelt, die von der eingangs genannten Elektrofixierung Gebrauch macht und im wesentlichen dadurch gekennzeichnet ist, daß man in einer Durchflußzelle mit permanenter Elektrodenanordnung arbeitet und die Abtrennung der Targetflüssigkeit unter Aufrechterhaltung der Fixierungsspannung vornimmt.

Weitere Besonderheiten der Erfindung ergeben sich aus den Patentansprüchen und der nachfolgenden Beschreibung.

Die erfindungsgemäße Technik erlaubt die glatte Einkopplung der Radionuklid/Targetflüssigkeits-Trennung in den Radiotracer-Syntheseprozeß bei bes. guter Wirtschaftlichkeit durch Minimierung von Targetverlusten bei hoher Nuklidausbeute und rascher Verfahrensweise, die einem automatischen Betrieb bestens angepaßt ist.

Diese Technik wurde ausgehend von einem zyklotron-bestrahlten H₂¹⁸O-Target entwickelt und daher nachfolgend im wesentlichen unter Bezugnahme auf dieses spezielle Beispiel beschrieben.

Das erfindungsgemäße Verfahren basiert auf folgendem Prinzip: Polarisierbare oder ionogene, trägerfreie Radionuklide, gelöst in Flüssigkeiten mit geringer elektrischer Leitfähigkeit, werden in der Weise aus der Lösung isoliert, daß in einem elektrischen Feld das jeweilige Radioisotop in polarisierter oder ionischer Form zu der entgegengesetzt geladenen Elektrode wandert und dort durch eine Art "Elektroadsorption" fixiert wird. Dabei erweist sich eine Koaxialgeometrie mit relativ großer Fixierungselektrode und die Vermeidung von Feldstärkespitzen als besonders zweckmäßig für die trägerfreie Aufsammlung des Radionuklids aus der von ionogenen Zusätzen freien Targetflüssigkeit. Nach erfolgter Fixierung des Radionuklids an der Elektrode kann die Targetflüssigkeit praktisch quantitativ vom Radionuklid - bei Aufrechterhaltung des elektrischen Feldes - entfernt werden. Durch nachfolgende Aufhebung des elektrischen Feldes oder kurzzeitige Umpolung der Elektroden kann das Radioisotopanion bzw. -kation wieder in Lösung gebracht werden, wofür insb. hochreines "normales" Wasser dient. Anschließend kann die on-line Verteilung der wäßrigen Aktivlösung zu den Syntheseboxen erfolgen.

Die erfindungsgemäße Durchflußzelle ist insbesondere direkt in den Transportweg von der Bestrahlungsstation zur Radiotracersynthese (z.B. Herstellung von [¹⁸F]-Fluordesoxyglucose) bzw. Syntheseapparatur eingeschaltet oder bildet ggf. sogar einen Teil der Transportleitung oder der Syntheseapparatur selbst.

Als Elektrodenmaterial kommen Stoffe in Betracht, die einerseits unter den gegebenen Bedingungen eine chemische Reaktion oder irreversible Insertion des Radionuklids in das Elektrodenmaterial ausschließen und andererseits das In-Lösung-Gehen des Radionuklids bei Umpolung des Systems gewährleisten. Glaskohlenstoff (Sigradur^{(R)}) sowie Platin erfüllen die Voraussetzungen. Vor der Ablösung des Radionuklids von der Elektrode kann - bei angelegtem Feld - zweckmäßigerweise "zwischengespült" werden, und das nachfolgende Inlösungbringen des Nuklids kann bei abgeschaltetem Feld ggf. unter Erwärmung erfolgen. Als Lösungsmittel kann hochreines Wasser oder ggf. das für die anschließende Synthese des Radiotracers vorgesehene Lösungsmittel (z.B. Acetonitril ggf. mit Zusätzen wie Lösungsvermittlern z.B. Komplexbildnern) dienen.
Als Spannungsquelle dient zweckmäßigerweise eine Gleichspannungsquelle, die stufenweise bis etwa 30 V Spannung abgibt. Für die Abscheidung werden Feldstärken angestrebt, die vorzugsweise nicht über 100 V/cm hinausgehen. Eine möglichst eingeebnete, "geschlossene" Oberfläche der Fixierungselektrode erscheint zweckmäßig.

Weitere Besonderheiten der Erfindung ergeben sich aus den nachfolgend wiedergegebenen Durchführungsbeispielen, an Hand der beigefügten Zeichnungen. Es zeigen im einzelnen:
- Figur 1: ein Schema für eine Trennapparatur mit Durchflußzelle;
- Figur 2: eine dafür geeignete Durchflußzelle im Detail;
- Figur 3: ein Schema für eine als Leitungsteil fungierende Durchflußzelle und
- Figur 4: ein Kurvenbild für ein Beispiel der Elektrofixierung und -wiederauflösung eines Radionuklids.

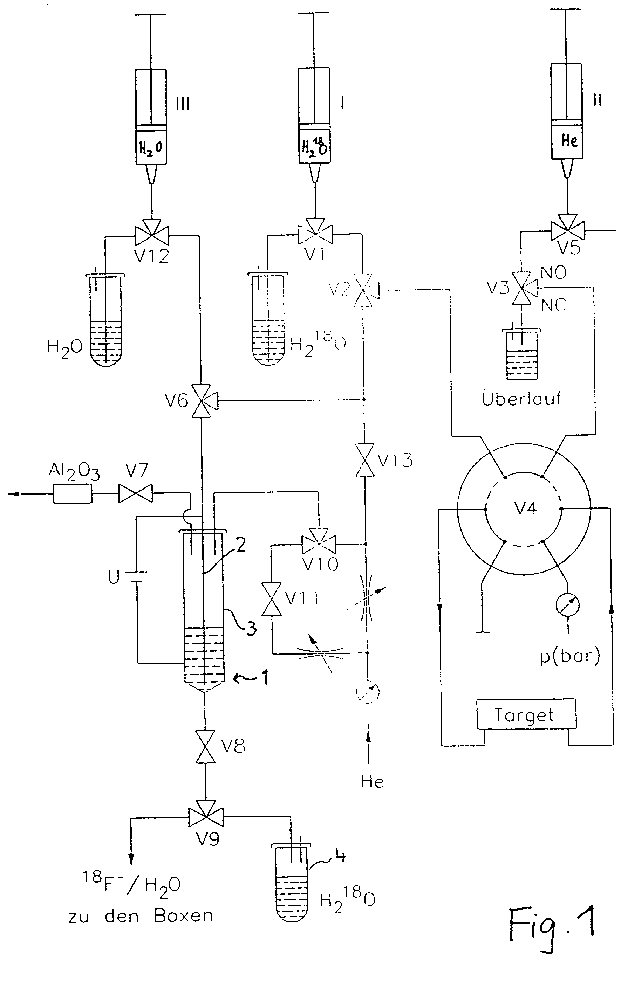

Gemäß Figur 1 umfaßt eine Anordnung für die Abtrennung von ¹⁸F aus zyklotron-bestrahltem H₂¹⁸O eine Durchflußzelle 1 mit einer als Zulaufkapillare ausgebildeten Elektrode 2 und einer die Gefäßwand bildenden, im wesentlichen zylindrischen Anode 3. Das Schema wird an Hand der nachfolgend angegebenen Verfahrensschritte der Radionuklid/Targetflüssigkeits-Trennung ohne weiteres verständlich werden:

### 1) Transport der ¹⁸F enthaltenden Targetflüssigkeit (H₂¹⁸O) von der Bestrahlungsstation (Target) zur Durchflußzelle.

Mittels der gasgefüllten (He) Spritze II wird die wäßrige Phase aus dem Target durch die Ventile V2 und V6 in die Durchflußzelle 1 gedrückt. Der kontrollierte, langsame Transport verhindert ein Verspritzen des am unteren Ende der Zelle über die Kapillare 2 zulaufenden ¹⁸O-Wassers innerhalb der Zelle.

### 2. Abscheidung des ¹⁸F durch Elektroadsorption.

Bei einer Gleichspannung von 20 V wird das negativ geladene Fluoridion an dem gegenüber der als Kathode geschalteten Kapillare 2 anodisch geschalteten, zylindrischen Sigradur^{(R)} Mantel 3 innerhalb von etwa 6 min abgeschieden bzw. fixiert. Nach der Elektroadsorption des ¹⁸F wird das von ionogenen Zusätzen freie ¹⁸O-Wasser unter Aufrechterhaltung des elektrischen Feldes mit einem schwachen, regelbaren Heliumstrom, der über die Ventile V11 und V10 zutritt, in das Sammelgefäß 4 transportiert. Das für die Bestrahlung eingesetzte Wasser wird so nahezu quantitativ zurückgewonnen.

### 3) Überführung des elektrofixierten ¹⁸F in "normales" Wasser.

In die Zelle 1 werden mittels der Spritze III über die Ventile V6 unf V12 2 ml Wasser eingefüllt. Zur raschen und quantitativen Überführung des ¹⁸F in die wäßrige

Phase wird das Wasser während der Umpolung des elektrischen Feldes (die Platinkapillare 2 bildet nunmehr die Anode) vorzugsweise erwärmt. Hierzu wird der Sigradur^{(R)} Mantel 3 durch eine Widerstandsheizung auf etwa 50°C gebracht. - Für den Ablösevorgang kann das für die Fixierung angelegte und während der Entfernung des H₂¹⁸O aus der Zelle und ggf. anschließender "Zwischenspülung" aufrechterhaltene Feld auch nur kurzzeitig umgepolt und dann abgeschaltet oder auch lediglich abgeschaltet werden. - Das ¹⁸F-haltige Wasser wird nach etwa 5 min mit Hilfe eines Heliumstromes über die Ventile V8 und V9 durch eine Polypropylenleitung zu den Synthesemodulen (Boxen) transportiert.

### 4) Trocknung der Zelle zur Vorbereitung des Systems für eine erneute Separation.

Die entleerte Zelle 1 wird - bei gleichzeitiger Erwärmung des Sigradur^{(R)} Mantels 3 - durch einen Heliumstrom getrocknet, damit eine erneute Befüllung der Zelle mit ¹⁸F/¹⁸O-Wasser nicht zu einer Verdünnung des Targetwassers und damit zu einer Qualitätsminderung desselben führt. Beim Trockenprozeß wird Helium über die Ventile V13 und V6 durch die Platinkapillare eingeleitet und über das Ventil V7 und die angekoppelte Al₂O₃-Kartusche nach außen abgeleitet. Die Kartusche hat den Zweck, evtl. gasförmig austretendes ¹⁸F zu fixieren und so eine Kontamination der Umgebung zu verhindern. Die Trocknung dauert etwa 15 min. (Das Ventil V7 ist bei jedem Füllvorgang und während der Elektroadsorption und -desorption geöffnet).

Die Schritte 1 bis 3 nehmen insgesamt etwa 15 min in Anspruch.

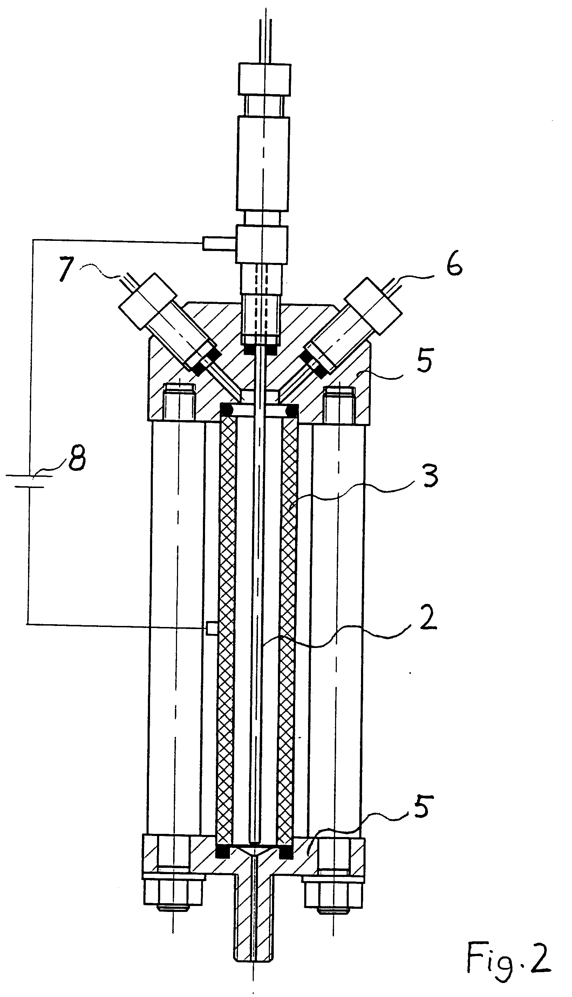

Gemäß Figur 2 besteht die Durchflußzelle im wesentlichen aus dem Mantel 3 und einer axialen Platinkapillare 2, durch die hindurch das zylinderförmige Gefäß gefüllt wird bzw. durch die Inertgas in die Kammer eingeleitet werden kann. Die Höhe des Zylinders ist so bemessen, daß der Füllstand der ¹⁸F/H₂¹⁸O-Lsg. etwa 50 - 70 % des Gesamtvolumens beträgt. Das Innenvolumen der Pt-Kapillare beträgt 35 µl. Bei normalem Füllstand ist das Flüssigkeitsvolumen innerhalb der Kapillare etwa 20 µl, so daß die der ¹⁸F-Fluoridabscheidung nicht zur Verfügung stehende Flüssigkeitsmenge lediglich 1 - 2 % des Gesamtvolumens entspricht. Das System wird durch eine Kunststoffhalterung 5 fixiert und abgedichtet. Am unteren Ende der Halterung ist ein flacher Trichter eingearbeitet, der in die das Wasser abführende Leitung einmündet. Im Kopfteil der aus PEEK gefertigten Halterung 5 befindet sich eine He-Zuleitung 6 sowie eine Öffnung 7, durch die Gas nach außen abgeleitet werden kann. Ein regelbarer Gasstrom ermöglicht eine kontrollierte Entleerung der Zelle. Der Sigradurzylinder 3 und die Platinkapillare sind an eine Gleichstromquelle 8 angeschlossen und können wahlweise als Kathode oder Anode geschaltet werden. Darüber hinaus kann die Durchflußzelle durch einen Warmluftstrom oder eine elektrische Heizung, wie z.B. eine Heizwicklung, erwärmt werden. Besonders zweckmäßig wird die Anordnung durch eine Programmsteuerung ergänzt, die für die Spannungsbeaufschlagung und Beheizung der Zelle sorgt.

Bei dem gezeigten Beispiel hatte der Sigradur^{(R)} Zylinder eine Länge von 70 mm und einen Innendurchmesser von 7 mm. Der Innendurchmesser der Platin-Kapillare betrug 0,8 mm und der Elektrodenabstand 2,7 mm.

Die vorstehend genannten Zahlenwerte sind nur Beispiele, die selbstverständlich gemäß den jeweiligen praktischen Erfordernissen abgewandelt werden können, wobei zu berücksichtigen ist, daß die Targetflüssigkeit bei batch-Betrieb in der Zelle zweckmäßigerweise unter Freilassung eines gewissen Puffervolumens unterzubringen ist und der Elektrodenabstand für den raschen Transport der Ionen zur Elektrode in Anbetracht von Aktivitätsverlusten bei Isotopen mit kurzer Halbwertzeit gering sein sollte.

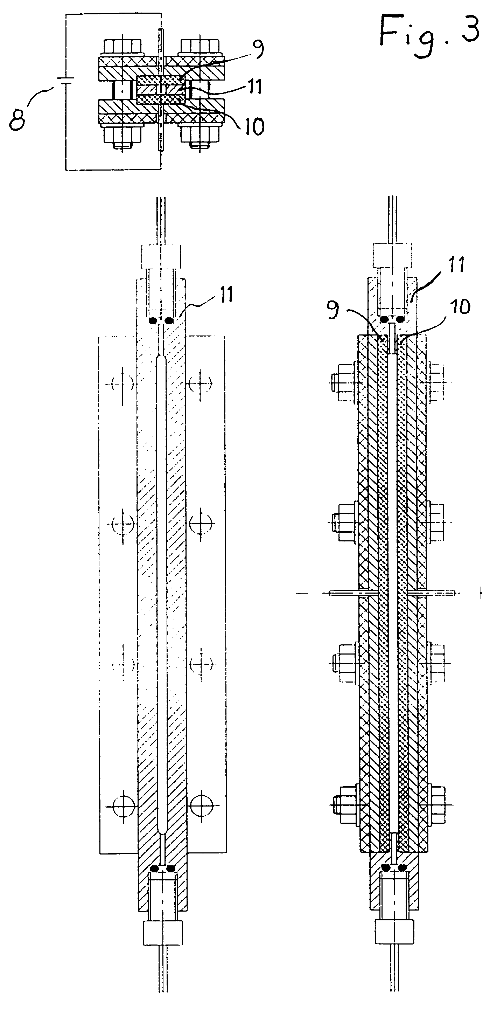

Eine alternative Durchflußzelle für die ¹⁸F/H₂¹⁸O-Separation wird in Figur 3 angedeutet. Sie umfaßt zwei parallel angeordnete Sigradurplatten 9, 10, die einen Abstand von etwa 2 mm haben. Entsprechend Figur 3 sind die als Elektroden fungierenden Platten durch einen umlaufenden Kunststoffstreifen 11 voneinander getrennt, der sowohl eine elektrische Isolation als auch eine Abdichtung des zentral angeordneten rechteckigen Kanals gewährleistet, durch den die ¹⁸F-Lsg. bei anliegendem Feld kontinuierlich hindurchgeleitet werden kann.

Nach der Elektrofixierung des ¹⁸F-Fluorids kann die Zelle mit einem organischen Solvens gespült und somit von restlichem Wasser befreit werden. Nach Umpolung kann in Gegenwart einer Lösung von Phasentransferkatalysator in organischem Solvens das Fluorid wasserfrei in die organische Phase überführt werden und steht somit - ohne thermische Entwässerung - für eine nukleophile Synthese von ¹⁸F-Radiopharmaka zur Verfügung, wie sie z.B. in der EP 0167103B1 angegeben ist.

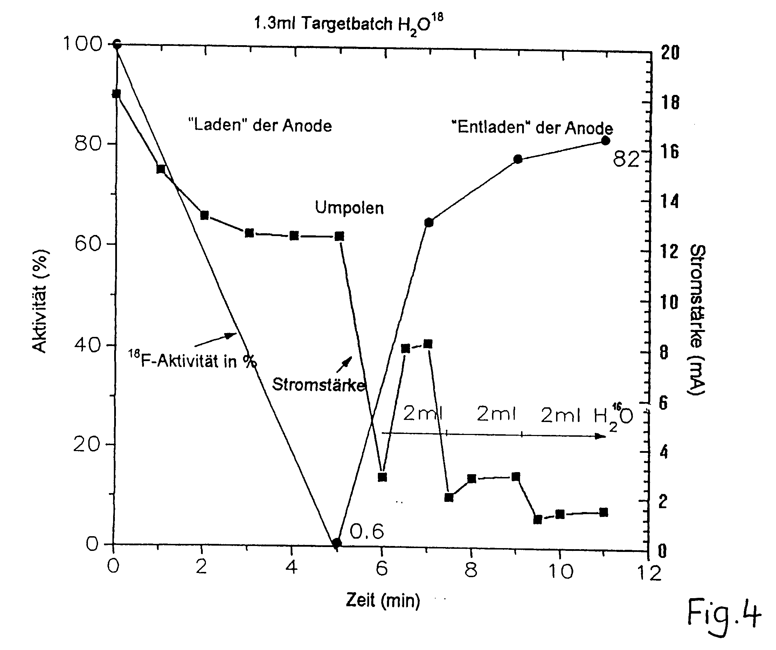

Das Diagramm Fig. 4 zeigt exemplarisch das Ergebnis der Abtrennung von ¹⁸F-Fluorid von H₂¹⁸O. Bei einer Spannung von 20 V wurde ¹⁸F quantitativ an der Sigraduranode fixiert. Im Verlauf der Abscheidung ist eine zeitabhängige Stromabnahme erkennbar, die nach etwa 5 min zum Stillstand kommt. Die Desorption des Radionuklids nach Umpolung des Feldes in Gegenwart von normalem Wasser wurde zeitabhängig gemessen. Die in diesem Fall nach 3maligem Wasserwechsel (je 2 ml) erzielte Aktivitätsausbeute von 82% entspricht nicht dem praktisch erreichbaren Ergebnis. Einmalige Wasserzugabe und entgegengesetzte Polung bei gleichzeitiger Erwärmung der Durchflußzelle führten zu einer 18F-Rückgewinnung von etwa 90%.

## Patentansprüche

1. Verfahren zur Abtrennung von trägerfreien Radionukliden aus flüssigem bzw. verflüssigbarem Targetmaterial mit geringer elektrischer Leitfähigkeit, die durch einen Nuklearprozeß, insbesondere durch Zyklotronstrahlung, in ionisierbarer bzw. polarisierbarer Form entstehen, innerhalb einer Elektrodenanordnung durch Anlegen eines elektrischen Feldes zur Elektrofixierung des Nuklids an einer der Elektroden, von der das Nuklid nach Entfernung der Targetflüssigkeit und Zugabe von Lösungsmittel für die weitere Verarbeitung bei abgeschaltetem Feld oder in einem Feld entgegengesetzter Polung wieder in Lösung gebracht wird,
**dadurch gekennzeichnet,**
daß man in einer Durchflußzelle mit permanenter Elektrodenanordnung arbeitet und die Abtrennung der Targetflüssigkeit unter Aufrechterhaltung der Fixierungsspannung vornimmt.

2. Verfahren nach Anspruch 1,
**dadurch gekennzeichnet,**
daß man eine Elektrodenanordnung mit porenfreier inerter Oberfläche, insbesondere aus Glaskohlenstoff oder Edelmetall, insbesondere Platin, wählt.

3. Verfahren nach Anspruch 2,
**dadurch gekennzeichnet,**
daß man als Elektrode für die Elektrofixierung des Radionuklids die Gefäßwand wählt.

4. Verfahren nach Anspruch 3,
**dadurch gekennzeichnet,**
daß die Elektrofixierung des Radionuklids in einem koaxialen Feld mit Anreicherung des Radionuklids an der zylindrischen Außenelektrode erfolgt.

5. Verfahren nach Anspruch 4,
**dadurch gekennzeichnet,**
daß die Flüssigkeitszufuhr zur Zelle über die als Kapillare ausgebildete axiale Elektrode erfolgt.

6. Verfahren nach einem der vorangehenden Ansprüche,
**dadurch gekennzeichnet,**
daß die Elektrofixierung des Radionuklids bei Feldstärken von 10 - 100 V/cm erfolgt.

7. Verfahren nach einem der vorangehenden Ansprüche,
**dadurch gekennzeichnet,**
daß mit Fixierungselektrodenflächen von ≥ 3 cm² gearbeitet wird.

8. Verfahren nach einem der vorangehenden Ansprüche,
**dadurch gekennzeichnet,**
daß die Ablösung des Nuklids von der Elektrode unter Erwärmung erfolgt.

9. Verfahren nach einem der vorangehenden Ansprüche
**dadurch gekennzeichnet,**
daß die Flüssigkeit aus der Durchflußzelle durch Inertgas, insb. Helium, ausgetrieben wird.

10. Verfahren nach einem der vorangehenden Ansprüche,
**dadurch gekennzeichnet,**
daß die Durchflußzelle durch die Transportleitung von der Bestrahlungsstation zur Markierungsapparatur oder Teile derselben oder durch das Reaktionsgefäß der Markierungsapparatur gebildet wird.

11. Verwendung des Verfahrens nach einem der vorangehenden Ansprüche für die Radioisotop-, insbesondere ¹⁸F-Markierung von organischen Verbindungen durch nukleophilen Austausch.

12. Durchflußzelle zur Durchführung des Verfahrens nach einem der vorangehenden Ansprüche,
**gekennzeichnet durch**
einen Glaskohlenstoffzylinder (3) als Außenwand und Fixierungselektrode und eine über dessen Gesamtlänge reichende Platinkapillare (2) als Gegenelektrode, der über einen flachen Konus mit geringer Steigung in einer kurzen zu einem Ventil führenden Kapillare endet und am oberen Ende durch eine die Gegenelektrode halternde Kappe mit Helium-Anschluß (6) und Druckausgleichsöffnung (7) abgeschlossen wird.

13. Durchflußzelle nach Anspruch 12,
**gekennzeichnet durch**
eine Heizwicklung auf der Zylinderaußenwand sowie eine Steuerung für die Spannungsversorgung und Beheizung der Zelle.

## Claims

1. A process for separating carrier-free radionuclides from liquid or liquefiable target material with a low electrical conductivity, which radionuclides are formed in ionisable or polarisable form by a nuclear process, particularly by cyclotron radiation, inside an electrode arrangement by the application of an electrical field for the electrofixation of the nuclide to one of the electrodes, from which, after removing the target liquid and adding solvent, the nuclide is brought into solution again for further processing with the field switched off or in a field of opposite polarity,
characterised in that
a flow cell having a permanent electrode arrangement is employed, and separation of the target liquid is effected whilst maintaining the fixing voltage.

2. A process according to claim 1,
characterised in that
an electrode arrangement is selected which has a pore-free inert surface, particularly of glassy carbon or a noble metal, especially platinum.

3. A process according to claim 2,
characterised in that
the vessel wall is selected as the electrode for the electrofixation of the radionuclide.

4. A process according to claim 3,
characterised in that
electrofixation of the radionuclide is effected in a coaxial field with enrichment of the radionuclide at the cylindrical outer electrode.

5. A process according to claim 4,
characterised in that
the liquid is fed to the cell via the axial electrode, which is constructed as a capillary.

6. A process according to any one of the preceding claims,
characterised in that
electrofixation of the radionuclide is effected at field strengths of 10 - 100 V/cm.

7. A process according to any one of the preceding claims,
characterised in that
fixing electrode area of ≥ 3 cm² are employed.

8. A process according to any one of the preceding claims,
characterised in that
removal of the nuclide from the electrode is effected whilst heating.

9. A process according to any one of the preceding claims,
characterised in that
the liquid is expelled from the flow cell by inert gas, particularly by helium.

10. A process according to any one of the preceding claims,
characterised in that
the flow cell is formed by the transport line from the radiation station to the labelling apparatus or by parts of the same, or by the reaction vessel of the labelling apparatus.

11. The use of the process according to any one of the preceding claims for the labelling of organic compounds with radioisotopes, particularly for labelling with ¹⁸F, by nucleophilic exchange.

12. A flow cell for carrying out the process according to any one of the preceding claims,
characterised by
a glassy carbon cylinder (3) as the outer wall and fixing electrode and by a platinum capillary (2) extending over the entire length thereof as a counterelectrode, which cylinder terminates, via a shallow cone of slight gradient, in a short capillary which leads to a valve, and which is closed at its top end by a cap which supports the counterelectrode and which comprises a helium connection (6) and a pressure equalisation opening (7).

13. A flow cell according to claim 12,
characterised by
a heater winding on the outer wall of the cylinder and by a control system for the voltage supply to and the heating of the cell.

## Revendications

1. Procédé pour séparer des radionuclides sans entraîneurs à partir d'un matériau cible liquide ou pouvant être liquéfié, possédant une conductibilité électrique faible et qui, sous l'effet d'un processus nucléaire, notamment sous l'effet d'un rayonnement cyclotron, apparaît sous une forme pouvant être ionisée ou polarisée, à l'intérieur d'un dispositif d'électrodes par application d'un champ électrique pour l'électrofixation du nuclide sur l'une des électrodes, à partir de laquelle le nuclide est amené à nouveau en solution, après élimination du liquide cible et addition d'un solvant pour le traitement ultérieur, avec suppression du champ ou dans un champ ayant une polarité opposée, caractérisé en ce qu'on travaille dans une cellule à traversée avec un dispositif d'électrodes permanent et on réalise la séparation du liquide cible en maintenant la tension de fixation.

2. Procédé selon la revendication 1, caractérisé en ce qu'on choisit un dispositif d'électrodes possédant une surface perforée, notamment en verre-carbone ou en métal précieux, notamment le platine.

3. Procédé selon la revendication 2, caractérisé en ce qu'on choisit la paroi du récipient en tant qu'électrode pour l'électrofixation du radionuclide.

4. Procédé selon la revendication 3, caractérisé en ce que l'électrofixation du radionuclide s'effectue dans un champ coaxial avec enrichissement du radionuclide sur l'électrode cylindrique extérieure.

5. Procédé selon la revendication 4, caractérisé en ce que l'amenée du liquide à la cellule s'effectue par l'intermédiaire de l'électrode axiale agencée sous la forme d'un capillaire.

6. Procédé selon l'une des revendications précédentes, caractérisé en ce que l'électrofixation du radionuclide s'effectue avec des intensités de champ de 10-100 V/cm.

7. Procédé selon l'une des revendications précédentes, caractérisé en ce qu'on travaille avec des surfaces de l'électrode de fixation ≥ 3 cm².

8. Procédé selon l'une des revendications précédentes, caractérisé en ce que la dissolution du nuclide est exécutée par l'électrode moyennant un chauffage.

9. Procédé selon l'une des revendications précédentes, caractérisé en ce que le liquide est extrait de la cellule à traversée au moyen d'un gaz inerte, notamment de l'hélium.

10. Procédé selon l'une des revendications précédentes, caractérisé en ce que la cellule à traversée est formée par la canalisation de transport s'étendant depuis le poste d'irradiation à l'appareil de marquage ou des parties de ce dernier ou bien par l'enceinte réactionnelle de l'appareil de marquage.

11. Utilisation du procédé selon l'une des revendications précédentes pour le marquage de composés organiques par radio-isotopes, notamment le ¹⁸F, au moyen d'un échange nucléophile.

12. Cellule à traversée pour la mise en oeuvre du procédé selon l'une des revendications précédentes, caractérisée par un cylindre en verre-carbone (3) utilisé en tant que paroi extérieure et électrode de fixation et un capillaire en platine (2), utilisée comme contre-électrode et qui s'étend sur toute la longueur du cylindre, cylindre qui se termine, par l'intermédiaire d'un cône plat ayant une faible pente, par un capillaire court aboutissant à une soupape et est fermé, à son extrémité supérieure, par un capuchon retenant la contre-électrode et pourvu d'un raccord (6) pour l'hélium et d'une ouverture de compensation de pression (7).

13. Cellule à traversée selon la revendication 12, caractérisée par un enroulement de chauffage disposé sur la paroi extérieure du cylindre ainsi que par une unité de commande pour l'alimentation en tension et le chauffage de la cellule.
